# EUROPEAN PATENT SPECIFICATION

(11) **EP 4 552 569 B1**
(45) Date of publication and mention of the grant of the patent: **24.06.2026**
(21) Application number: 23208631.4
(22) Date of filing: 08.11.2023
(51) Int. Cl.: A61B 5/1455, A61B 5/18, B60W 40/08, G06V 20/59

(54) **MIND WANDERING DETECTION DEVICE, SYSTEM AND METHOD**
VORRICHTUNG, SYSTEM UND VERFAHREN ZUR ERKENNUNG VON KONZENTRATIONSDEFIZIT
DISPOSITIF, SYSTÈME ET PROCÉDÉ DE DÉTECTION D'INATTENTION

(43) Date of publication of application: 14.05.2025
(73) Proprietor: TOYOTA JIDOSHA KABUSHIKI KAISHA, Toyota-shi, Aichi-ken, 471-8571 (JP); UCL Business Ltd, London WC1E 6BT (GB)
(72) Inventor: AMBECK-MADSEN, Jonas, 1140 BRUSSELS (BE); LAVIE, Nilli, LONDON, WC1E 6BT (GB); BRUCKMAIER, Merit, LONDON, WC1E 6BT (GB); TACHTSIDIS, Ilias, LONDON, WC1E 6BT (GB); ALBRECHT, Vivien, LONDON, WC1E 6BT (GB)
(74) Representative: Cabinet Beau de Loménie

(56) References cited:
- WO-A1-2019/223880
- WO-A1-2020/182281
- US-A1- 2020 029 880

## Description

### BACKGROUND OF THE INVENTION

### 1. Field of the invention

The present disclosure relates to the field of safety in technical applications, and more particularly to a mind wandering detection device and mind wandering detection method for a technical system. The device and method may find applications in all kinds of industries, including the automotive industry. For instance, the device and method may be used to detect undesirable mind wandering of an operator of a technical system such as a vehicle, a robot or a manufacturing apparatus, in order to warn the operator or otherwise take this mind wandering into account to prevent harm to themselves or third-parties.

### 2. Description of Related Art

WO 2019/223880, by the Applicants, relates to a system and method for determining the perceptual load and the level of stimulus perception of a human brain. This document tackles the issue of identifying a type of "inattentional blindness" where participants entirely fail to perceive a stimulus due to the lack of perceptual resources. Differently, mind wandering can act as a form of internal distraction to operators of technical systems such as vehicles, robots or manufacturing apparatuses, diverting their attention from the task they are assigned. In the example of driving, the driver's attention may be focused on distracting thoughts unrelated to the driving scene and this can lead to inattention with regard to their driving task. Moreover, such inattention can occur without any change in their gaze direction so that the driver's eyes are still looking at the road as before but they fail to see what is in front of them due to "inattentional blindness". This can result in safety-critical information being missed. These effects of driver inattention are known to be a major cause of accidents (e.g., car and aeroplane crashes or industrial accidents).

It is therefore desirable to have a portable, non-invasive device that can detect the level of mind wandering, especially considering that mind wandering is an internal state without clear external indicators. Specifically, it is desirable to have a non-invasive device detecting mind wandering without relying on differences in eye movement or position patterns, without being sensitive to operator's motion as electroencephalography (EEG) would be.

Finally, not all mind wandering is necessarily distracting - some mind wandering can be 'harmless' and may continue in the background without causing any task interference, while other types of mind wandering can be highly distracting and take attention away from the driving or operating task. It is therefore important to have a device that is able to detect mind wandering that acts as attention distraction (hereinafter "undesirable mind wandering"), rather than all mind wandering indiscriminately.

### SUMMARY

In this respect, the present disclosure relates to a mind wandering detection device for a technical system such as a vehicle, a robot or a manufacturing apparatus, configured to:
- obtain a neural marker signal representing a level of a metabolic neural marker of mind wandering of an operator of the technical system;
- obtain a perceptual load signal representing a level of perceptual load of the operator;
- determine that the operator has undesirable mind wandering based on the neural marker signal and the perceptual load signal.

The mind wandering detection device is configured to obtain signals and process them in order to output a determination of undesirable mind wandering. The mind wandering detection device may have the hardware structure of a computer.

The technical system may be a vehicle, a robot, a manufacturing apparatus or any other technical system. The operator may be any human being whose attention is required for proper operation of the technical system, e.g. a human being at least partially controlling or monitoring the technical system. For instance, an operator of a vehicle may be the (in-vehicle or remote) driver of the vehicle, even if operation of the vehicle is shared between the driver and an autonomous driving controller.

A metabolic neural marker is a biological indicator of neurometabolic changes in the operator's brain, as opposed e.g. to electrophysiological markers (as measured e.g. by electroencephalography (EEG) or magnetoencephalography (MEG)) and haemodynamic markers (as measured e.g. by functional magnetic resonance imaging (fMRI) or functional near-infrared spectroscopy (fNIRS)). The metabolic neural marker may be a marker of cellular metabolism in the operator's brain. The metabolic neural marker is a marker which detects a type of mind wandering that a fully attending person does not engage in.

The neural marker signal may reflect the evolution over time of the metabolic neural marker for the operator or the level of the metabolic neural marker at a given time.

Perceptual load is a measure of the operator's mental processing demand that a given task imposes (e.g. operating the technical system). In an example, the perceptual load may be a visual load, however other senses such as hearing, smelling, touching may be taken into account as alternatives or in addition thereto. For instance, a complex visual environment with various shapes and colors is an example of a high perceptual load, whereas a relatively empty and unchanging visual environment is an example of a low perceptual load. In another example, the same environment can form a relatively high perceptual load if the operator is expected to extract much information therefrom, and a relatively low perceptual load if the operator is expected to extract only partial information therefrom.

The perceptual load signal may reflect the evolution over time of the operator's perceptual load or the level of the operator's perceptual load at a given time, desirably about the same time as the time that the neural marker signal corresponds to. For instance, the neural marker signal and the perceptual load signal may be obtained for the same time window, or at least overlapping time windows.

According to the present disclosure, whether mind wandering is harmless or undesirable depends on the operator's perceptual load. In other words, mind wandering is affected by attention demand in the task. Specifically, the inventors suggest that there is a bi-directional trade-off relationship between neural activity related to mind wandering versus perception: increased demands on neural activity related to perceptual processing in an attended task are associated with reduced neural activity related to any unattended processing, including mind wandering, and conversely, periods of mind wandering result in disengagement of attention from perceptual processing and thus result in reduced neural activity related to perception. Thus, in obtaining both the neural marker signal and the perceptual load signal, the mind wandering detection device is able not only to detect mind wandering but more precisely to detect undesirable mind wandering, namely mind wandering which prevents the operator from focusing on their technical task.

Besides, the measure of a metabolic neural marker is directly related to the operator's neural response, which ensures greater reliability and faster response of the mind wandering detection. Indeed, metabolic functions work at a faster time scale than other types of responses such as haemodynamic functions. Thus, in order to determine whether an operator engages in undesirable mind wandering, the proposed mind wandering detection device judiciously combines the different fields of biomedical optical physics/engineering and cognitive neuroscience of attention.

Optionally, the mind wandering detection device is configured to determine that the operator has undesirable mind wandering when the level of the neural marker is greater than or equal to a first threshold and the level of perceptual load is greater than or equal to a second threshold. The thresholds may be set be the skilled person based on the signal ranges and experiments quantifying a relatively low vs. relatively high level of the neural marker, and likewise a relatively low vs. relatively high level of perceptual load.

Owing to those features, the mind wandering detection device has simple criteria for determining undesirable mind wandering and can be implemented robustly and efficiently.

Optionally, the mind wandering detection device is further configured to output a warning signal. For instance, the mind wandering detection device may output a warning signal when it has been determined that the operator has undesirable mind wandering. The warning signal may be a visual signal, a sound signal, a haptic signal or the like, or any combination thereof. In these embodiments, the mind wandering detection device enables to warn the operator and/or third-parties that undesirable mind wandering is occurring, and possibly stops the undesirable mind wandering by drawing the operator's attention, thus prompting them to focus back on their task.

Optionally, the mind wandering detection device is configured to output the warning signal if the undesirable mind wandering has been determined to last over a period longer than a preset period. In these embodiments, short undesirable mind wandering is ignored. Besides, if the undesirable mind wandering does not last over a period longer than the preset period, it means that the operator's mind wandering has stopped or is no longer undesirable. Therefore, unnecessary warnings are not sent in order to increase the operator's safety by not overloading them with information. In an example, the preset period may be between 2s-10s, with an option of gradually reducing the intervals if undesirable mind wandering is persistently detected. Finally, immediate warning signals may also be sent should perceptual load suddenly increase during an interval where undesirable mind wandering is detected, demanding the operator immediate attention.

Optionally, the metabolic neural marker is an oxidation state of cytochrome-c-oxidase enzyme. The oxidation state of cytochrome-c-oxidase enzyme, hereinafter shortened as oxCCO, is an intracellular marker. The oxCCO is one of the markers which underlie the mental processing of task-unrelated thoughts, in particular while the operator is required to focus attention on a visual task. The oxCCO is an example of how to directly measure brain tissue metabolism through quantification of mitochondrial functions (the cytochrome-c-oxidase being a mitochondrial enzyme indicative of cellular oxygen metabolism). The oxCCO is an example of a neural marker of mind wandering that is shown to be attenuated or even eliminated when people are attentive in a high load (more demanding) task. The oxCCO provides a reliable and fast indication of the operator's level of mind wandering.

Optionally, the neural marker is a marker within a pre-frontal cortex of the operator, optionally within the medial pre-frontal cortex of the operator. In these embodiments, contrary to expectations, the neural marker of the effects of perceptual load is not to be sought in the operator's visual cortex. The level of a neural marker such as oxCCO in the visual cortex is rather linked to presence of visual stimulation than occurrence of mind wandering.

The present disclosure is further directed to a mind wandering detection system, comprising the above-described mind wandering detection device, a neural marker sensor device configured to acquire the level of the metabolic neural marker of mind wandering and to output the neural marker signal to the mind wandering detection device, and a perceptual load sensor device configured to acquire the level of perceptual load and to output the perceptual load signal to the mind wandering detection device.

The mind wandering detection device may have any of the above-mentioned features. As a consequence, the neural marker sensor device may be configured to acquire the level of oxCCO and/or acquire the level of the neural marker in the operator's pre-frontal cortex.

The mind wandering detection system may be portable, for instance integrated to the technical system (e.g. vehicle, robot or manufacturing apparatus), or even wearable, i.e. worn by the operator. Alternatively, the neural marker sensor device and/or the perceptual load sensor device may be wearable and connected or synchronized to other parts of the mind wandering detection system, which may be integrated to the technical system. Therefore, the mind wandering detection system can be easily applied to in-situ applications, as opposed to bulky detectors found in labs and medical facilities.

Optionally, the neural marker sensor device comprises a broadband near infrared spectroscope. Broadband near-infrared spectroscopy (hereinafter shortened as BNIRS) is a type of near-infrared spectroscopy which performs spectroscopy based on broadband light, e.g. more than 100 wavelengths, preferably 120 wavelengths or more, in the near infrared domain (e.g. 650-900 nm, preferably 780-900 nm). In contrast, conventional functional near-infrared spectroscopy (fNIRS) only uses a couple of wavelengths, generally two or three, and cannot be used to accurately monitor metabolic neural markers such as oxCCO. While conventional fNIRS enables only to look at haemodynamic function, BNIRS gives access to metabolic functional changes. BNIRS enables to monitor metabolic markers which respond much faster to the operator's state of mind changes, whereas haemodynamic markers, accessible through conventional fNIRS, are a much more indirect and slower measure of the operator's mind wandering. The broadband near infrared spectroscope may comprise a spectroscope per se as well as the desired auxiliary systems to perform the desired BNIRS.

The broadband near infrared spectroscope may be configured to detect the changes in the absorption spectra of the oxidized and non-oxidized cytochrome-c-oxidase enzyme in the near infrared region.

Optionally, the perceptual load sensor device comprises a camera configured to cover a field of view of the operator and a processor connected to the camera to receive at least one image from the camera and compute the level of perceptual load from said at least one image. Covering the operator's field of view means that the camera has a field of view which corresponds to what an average operator would see in normal operating conditions. Therefore, it is not necessary to adapt the camera to each operator; the field of view can be estimated and/or the camera may have a broader field of view than the operator.

Computing the level of perceptual load may be carried out by image or video analysis techniques known per se in the art (e.g. detecting the density of other vehicles on a road, quantifying how much the environment changes, assessing computer vision machine-learned perceptual load features, etc.). Other factors may be taken into account to assess the level of perceptual load, such as how noisy the operator's environment is.

The present disclosure is further directed to a vehicle comprising the above-described mind wandering detection device or the above-described mind wandering detection system.

The present disclosure is further directed to a computer-implemented mind wandering detection method, comprising:
- obtaining a neural marker signal representing a level of a metabolic neural marker of mind wandering of an operator of a technical system such as a vehicle, a robot or a manufacturing apparatus ;
- obtaining a perceptual load signal representing a level of perceptual load of the operator;
- determining that the operator has undesirable mind wandering based on the neural marker signal and the perceptual load signal.

The mind wandering detection method may be carried out by the above-described mind wandering detection device or mind wandering detection system, and may have any feature corresponding to those detailed with respect to said device or system.

Optionally, the method is executed in real-time. That is, the method is executed without any waiting, as the neural marker signal and the perceptual load signal are being obtained. When the signals are obtained in a discrete manner, the sampling rate is high enough to accurately convey the evolution of the level of the metabolic neural marker and the level of perceptual load. Thus, signals obtained and mind wandering determined in real-time for a given time step are regarded as valid, with reasonable uncertainty, until the subsequent time step.

Thanks to the level of metabolic neural marker being a fast indicator of the operator's mind wandering, real-time application is possible, possibly without relying on other detections such as pupil diameter and the like, which are not as responsive and more indirect. In addition, real-time countermeasures, such as warning the operator, may be taken appropriately and timely.

The present disclosure is further related to a computer program set including instructions for executing the steps of the above-described mind wandering detection method when said program set is executed by at least one computer. This program set, comprising one or more programs, can use any programming language and take the form of source code, object code or a code intermediate between source code and object code, such as a partially compiled form, or any other desirable form.

The present disclosure is further related to a recording medium readable by at least one computer and having recorded thereon at least one computer program including instructions for executing the steps of the above-described mind wandering detection method. The recording medium can be any entity or device capable of storing the program. For example, the medium can include a mass memory device, such as a hard drive. In general, mass memory devices suitable for tangibly embodying computer program instructions and data include all forms of nonvolatile memory, including by way of example semiconductor memory devices, such as solid state storage (SSS), EPROM, EEPROM, and flash memory devices; magnetic disks such as internal hard disks and removable disks; magneto-optical disks.

Alternatively, the recording medium can be an integrated circuit in which the program is incorporated, the circuit being adapted to execute the method in question or to be used in its execution.

The present disclosure is further directed to a mind wandering detection device, configured to: obtain a neural marker signal representing a level of a neural marker of mind wandering of a subject; obtain a perceptual load signal representing a level of perceptual load of the subject; determine that the subject has undesirable mind wandering based on the neural marker signal and the perceptual load signal.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:

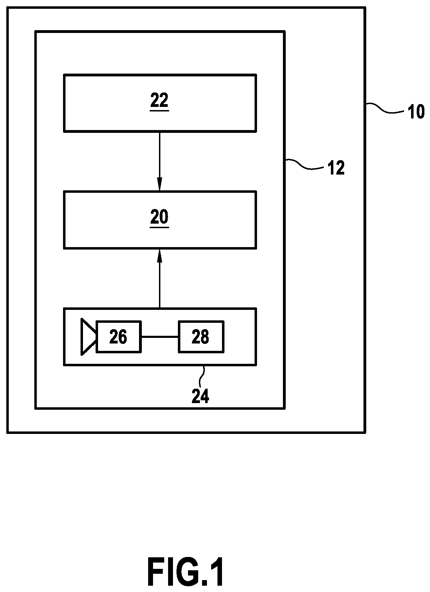

- Fig. 1 is a block diagram showing a vehicle, a mind wandering detection system and a mind wandering detection device according to an embodiment.

### DETAILED DESCRIPTION OF EMBODIMENTS

Figure 1 is a block diagram illustrating a technical system for an operator to operate. In the following, for conciseness, the technical system is assumed to be a vehicle 10 without loss of generality. Other technical systems are encompassed, including but not limited to a robot, a manufacturing apparatus, etc.

The vehicle 10 comprises subsystems and devices, such as a body, wheels, a drive train, a controller, etc., to perform its expected functions, e.g. transporting passengers and/or loads. These subsystems and devices are known per se to the person skilled in the art of the relevant type of vehicles and are omitted in the present disclosure.

In order to detect undesirable mind wandering of an operator (e.g. a driver), the vehicle 10 comprises a mind wandering detection system 12. The mind wandering detection system 12 comprises a mind wandering detection device 20, a neural marker sensor device 22 and a perceptual load sensor device 24. However, alternatively, the mind wandering detection system 12 may not be located within the vehicle 10 but anywhere, including remotely, where the mind wandering detection system 12 is able to monitor an operator of the vehicle 10 and detect undesirable mind wandering of that operator.

The mind wandering detection system 12 and/or the mind wandering detection device 20 may comprise an electronic circuit, a processor (shared, dedicated, or group), a combinational logic circuit, a memory that executes one or more software programs, and/or other suitable components that provide the described functionality. In other words, the mind wandering detection system 12 and/or the mind wandering detection device 20 may be a computer device. The mind wandering detection system 12 and/or the mind wandering detection device 20 may be connected to a memory, which may store data, e.g. a computer program which when executed, carries out the mind wandering detection method according to the present disclosure.

The mind wandering detection device 20, the neural marker sensor device 22 and the perceptual load sensor device 24 may be partly or fully implemented as software running on the mind wandering detection system 12 and/or as hardware elements of the mind wandering detection system 12.

As mentioned before, the mind wandering detection device 20 is configured to: obtain a neural marker signal representing a level of a metabolic neural marker of mind wandering of an operator of the technical system; obtain a perceptual load signal representing a level of perceptual load of the operator; and determine that the operator has undesirable mind wandering based on the neural marker signal and the perceptual load signal. The neural marker signal may be obtained from the neural marker sensor device 22. The perceptual load signal may be obtained from the perceptual load sensor device 24. The neural marker sensor device 22 and the perceptual load sensor device 24 may communicate with the mind wandering detection device 20, for transmission of said signals, in any desired manner.

Thus, the mind wandering detection device 20 is suitable for carrying out the above described mind wandering detection method. In particular, the mind wandering detection method may be executed in real-time: the neural marker sensor device 22 and the perceptual load sensor device 24 may transmit the neural marker signal and the perceptual load signal as soon as they are acquired, and the mind wandering detection device 20 may process those signals as soon as they are received to determine whether the operator has undesirable mind wandering. Alternatively, the neural marker signal and the perceptual load signal may be acquired beforehand, and obtained by the mind wandering detection device 20 later, e.g. in case of post-processing of the recorded monitoring.

Since the neural marker signal represents a level of a metabolic neural marker of mind wandering, which is a direct response of the operator's brain as opposed to haemodynamic signals and the like, real-time application can be envisaged because the neural marker signal more rapidly reflects the metabolic changes in the operator's brain. As an illustration, the typical responsiveness of metabolic neural markers is about 1 second, whereas the typical responsiveness of haemodynamic markers is about 20 seconds.

The neural marker sensor device 22 is configured to acquire a level of a metabolic neural marker of mind wandering for the operator of the vehicle 10 and to output a neural marker signal, representing said level, to the mind wandering detection device 20. For instance, the neural marker sensor device 22 may be configured to optically monitor the operator's brain to acquire the level of the desired metabolic neural marker of mind wandering.

In an embodiment, the neural marker sensor device 22 comprises a broadband near infrared spectroscope. Thus, as mentioned above, the neural marker sensor device 22 is configured to perform spectroscopy based on broadband light, which gives access to information not accessible by conventional techniques such as functional NIRS (fNIRS).

In an example, the neural marker sensor device 22 comprises a plurality of optodes (i.e. the optical equivalent of electrodes, e.g. optical fibers) to be placed over the operator's brain, e.g. the operator's pre-frontal cortex or specifically medial pre-frontal cortex (mPFC). The optodes may be fixed to a band or helmet fixed on the operator's head. For instance, an array carrying the optodes may be placed over the frontal cortex approximately 6 cm above the operator's nasion. More generally, the neural marker sensor device 22 may be portable.

The neural marker sensor device 22 may comprise at least one source optode, e.g. 4 source optodes, and at least one detector optode, e.g. 10 detector optodes. The detector optodes measure the spectrum of the light emitted by the source optodes and reflected back through the operator's scalp. The detector optodes may be arranged in at least one row, e.g. two rows of five detectors optodes with the source optodes in between. This may result in a plurality of measurement channels, e.g. 16 measurement channels. More details about the implementation of a BNIRS detector may be found in M. Bruckmaier, I. Tachtsidis, P. Phan, N. Lavie, Attention and Capacity Limits in Perception: A Cellular Metabolism Account. J. Neurosci. 40, 6801-6811 (2020).

BNIRS uses a broadband light spectrum, e.g. in the wavelength range from 650 nm to 950 nm (nanometer). BNIRS preferably simultaneously uses 120 wavelengths from 780 to 900 nm to resolve oxCCO. The attenuation data measured between 780 to 900 nm may be transformed into chromophore concentration changes (oxCCO) based on the Beer-Lambert law, assuming a differential pathlength factor of 6.26 and its wavelength dependence (P. Phan, et al., Multi-channel multi-distance broadband near-infrared spectroscopy system to measure the spatial response of cellular oxygen metabolism and tissue oxygenation. Biomed. Opt. Express 7, 4424 (2016)). Since the streams are assumed to last for 1 to 10 minutes, low-frequency fluctuations in the data are likely to contain task-related information. Therefore, a lowpass filter may be applied with a cut-off frequency of e.g. 0.08 Hz, preserving low-frequency components of the signal. To remove low-frequency changes that are not induced by the periodicity of the long task blocks and reflect noise of various physiological or technological origin, the data may be detrended by subtracting a linear fit between the mean of the e.g. 10 seconds preceding and following each stream. Motion artefacts may be corrected using a wavelet-based algorithm with an IQR parameter of 1.5 (B. Molavi, G. A. Dumont, Wavelet-based motion artifact removal for functional near-infrared spectroscopy. Physiol. Meas. 33, 259-270 (2012)). Since this algorithm could not correct for step-like motion artefacts (where the signal never returns to the same level as before the artefact vs. a spike-like motion artefact where the signal returns to the same level as pre-artefact), data may be inspected, e.g. manually, and the full stream may be excluded if it contains step-like motion artefacts, since this would affect the detrending procedure for the entire stream. This may be done on a channel-by-channel basis (given that each optode could undergo some movement independently of another). The neural marker sensor device may be calibrated for a given operator, e.g. by averaging the mean neural marker level across a given period (e.g. the 15 s interval) preceding each one of thought probes according to task condition (high vs. low) and thought-probe response (task focus vs. mind wandering). Therefore, the first threshold for the level of the neural marker may be set in an operator-dependent manner. To ensure that any effects found during the calibration are not driven by a few individuals disproportionally affecting the mean signals, and to identify the specific time periods of mind wandering, a bootstrap resampling analysis (1000 iterations, balanced; Efron and Tibshirani, 1993) can be run. In experiments, bootstrap analyses confirmed that all the significant main effects and interactions were significant for second by second comparisons (of mind wandering versus task focused) throughout the whole 15 seconds preceding a probe. In other words, a change in the mind wandering metabolic signal can be detected before a whole 15 second period. Bootstrapping can also detect longer periods than 15 seconds.

The neural marker sensor device 22 may be configured to acquire the level of oxidation state of cytochrome-c-oxidase enzyme (oxCCO), particularly in the operator's pre-frontal cortex or specifically medial pre-frontal cortex (mPFC). The oxCCO has been found to be a metabolic neural marker of mind wandering. More generally, the metabolic neural marker may be an intracellular marker of oxygen metabolism, since such marker is directly linked to neural energy consumption in response to neural firing and synaptic activity, and therefore provides a faster response to any change in the neural activity. Moreover, since the concentration of oxCCO does not change over relatively short time periods (e.g., hours), the ratio between oxidized and reduced CCO can be used to assess changes to the level of cellular metabolism.

If desired, the neural marker sensor device 22 may be further configured to acquire haemodynamic markers such as levels of blood oxygenation and blood oxygenation and deoxygenation (HbO2 and HHb), which give indirect - therefore delayed and potentially distorted - indications about the operator's neural response.

The perceptual load sensor device 24 is configured to acquire a level of perceptual load of the operator and to output a perceptual load signal, representing said level, to the mind wandering detection device 20. For instance, the perceptual load sensor device 24 may be configured to monitor stimulations (e.g. visual or sound stimulations) that impact the operator's perceptual load.

In an embodiment, the perceptual load sensor device 24 comprises a camera 26 configured to cover a field of view of the operator. The camera 26 may be aboard the vehicle 10. The camera 26 may be directed to a direction ahead of the vehicle 10. The camera 26 may have a range of view (e.g. height and width of the image) and/or a resolution comparable to the operator's field of view, e.g. of the same order of magnitude.

The perceptual load sensor device 24 may further comprise a processor 28 connected to the camera 26 to receive at least one image (e.g. an image, a series of images or a video) from the camera 26 and compute the level of perceptual load from said at least one image. The level of perceptual load may be derived from the cognitive content of the at least one image, such as the number, density, type, variety and/or movement of objects in the image. Image processing techniques, known per se in the field of computer vision, may be used by the processor to this end. Alternatively or in addition, the perceptual load of the operator may be estimated directly, e.g. as per the principles set out in the Applicants' international application WO 2020/182281.

Based on the neural marker signal and the perceptual load signal, the mind wandering detection device 20 is configured to determine whether the operator has undesirable mind wandering. That is, based on said signals, the mind wandering detection device 20 receives information representative of the levels of the above-mentioned metabolic neural marker and of the operator's perceptual load, and can determine not only whether mind wandering is detected but also whether that mind wandering is undesirable, i.e. whether the operator engages in mind wandering whereas the task they are assigned would require their full attention.

For instance, the mind wandering detection device 20 may be configured to determine that the operator has undesirable mind wandering when the level of the neural marker is greater than or equal to a first threshold and the level of perceptual load is greater than or equal to a second threshold. In other words, undesirable mind wandering may be detected when the level of oxCCO in the mPFC is high and when the operator's perceptual load is high.

Experiments conducted by the inventors assessed the level of mind wandering based on subjects' real-time report, while the subjects attended to a visual stream of rapidly changing cross shapes. Irregularly timed mind wandering probes queried the subjects whether they experienced task-unrelated thoughts in the period before the probes appeared. Subject pressed one key to indicate mind wandering or another key to indicate they were on task. Perceptual load was manipulated in the task by instructing people to monitor for either a simple color feature (low load - more generally, feature pop out search) or a combination of color and shape (high load - more generally, conjunction search). The metabolic neural marker levels were measured, here through BNIRS, and related to reports of mind wandering vs. 'on-task' thoughts under the different load conditions in the attended task. The results showed increased metabolism in frontal cortex during periods in which people reported mind wandering compared to no mind wandering when perceptual load was low. This metabolic signal was eliminated in high perceptual load.

A second experiment showed that the amount of detail in the task-unrelated thoughts, and thus detailed mind wandering, was significantly reduced when perceptual load was high (vs low). This indicates that the metabolic neural marker of mind wandering in the medial prefrontal cortex (mPFC) is specifically associated with the type of detailed mind wandering that predominantly occurs in low load conditions. If this neural marker shows high signal in high load task, this indicates the occurrence of mind wandering that an attentive person does not engage in under high perceptual load task situations. Therefore, the inventors identified a neural marker of mind wandering, e.g. oxCCO, that is shown to be attenuated or even eliminated when people are attentive in a high load (more demanding) task.

Low levels of oxCCO in the mPFC indicate that the operator does not engage in detailed mind wandering, irrespective of the level of perceptual load. When the level of oxCCO in the mPFC is high but the level of perceptual load is low, mind wandering does not affect the assigned task. This "harmless" mind wandering, as opposed to undesirable mind wandering, does not prevent the operator from being focused on the relatively undemanding task they are assigned to.

If desired, a calibration step may be carried out to determine the first threshold and/or the second threshold. Situations representative of the operator's operating tasks with the technical system (e.g. driving) or simulated situations as above may be used during the calibration step.

When undesirable mind wandering is detected, the mind wandering detection device 20 may output a warning signal to draw the operator's attention. In order to opportunely warn the operator, the warning signal may be output, e.g. only, if the undesirable mind wandering has been determined to last over a period longer than a preset period. The preset period may have a desired value that may depend on the application, e.g. 2s-10s, with an option of gradually reducing the intervals if undesirable mind wandering is persistently detected. Finally, immediate warning signals may also be sent should perceptual load suddenly increase during an interval where undesirable mind wandering is detected, demanding the operator immediate attention.

Although the present disclosure refers to specific exemplary embodiments, modifications may be provided to these examples without departing from the general scope of the invention as defined by the claims. For instance, instead of being located in a vehicle, the mind wandering detection system may be located at a remote facility for detecting undesirable mind wandering of a remote operator of the vehicle. Therefore, the description and the drawings should be considered in an illustrative rather than in a restrictive sense.

## Claims

1. A mind wandering detection device (20) for a technical system such as a vehicle (10), a robot or a manufacturing apparatus, configured to:
- obtain a neural marker signal representing a level of a metabolic neural marker of mind wandering of an operator of the technical system;
- obtain a perceptual load signal representing a level of perceptual load of the operator;
- determine that the operator has undesirable mind wandering based on the neural marker signal and the perceptual load signal.

2. The mind wandering detection device of claim 1, configured to determine that the operator has undesirable mind wandering when the level of the neural marker is greater than or equal to a first threshold and the level of perceptual load is greater than or equal to a second threshold.

3. The mind wandering detection device of claim 1 or 2, further configured to output a warning signal.

4. The mind wandering detection device of claim 3, configured to output the warning signal if the undesirable mind wandering has been determined to last over a period longer than a preset period.

5. The mind wandering detection device of any one of claims 1 to 4, wherein the metabolic neural marker is an oxidation state of cytochrome-c-oxidase enzyme.

6. The mind wandering detection device of any one of claims 1 to 5, wherein the neural marker is a marker within a pre-frontal cortex of the operator, optionally within the medial pre-frontal cortex of the operator.

7. A mind wandering detection system (12), comprising the mind wandering detection device (20) of any one of claims 1 to 6, a neural marker sensor device (22) configured to acquire the level of the metabolic neural marker of mind wandering and to output the neural marker signal to the mind wandering detection device (20), and a perceptual load sensor device (24) configured to acquire the level of perceptual load and to output the perceptual load signal to the mind wandering detection device (20).

8. The mind wandering detection system of claim 7, wherein the neural marker sensor device (22) comprises a broadband near infrared spectroscope.

9. The mind wandering detection system of claim 7 or 8, wherein the perceptual load sensor device (24) comprises a camera (26) configured to cover a field of view of the operator and a processor (28) connected to the camera (26) to receive at least one image from the camera (26) and compute the level of perceptual load from said at least one image.

10. A vehicle (10) comprising the mind wandering detection device (20) of any one of claims 1 to 6 or the mind wandering detection system (12) of any one of claims 7 to 9.

11. A computer-implemented mind wandering detection method, comprising:
- obtaining a neural marker signal representing a level of a metabolic neural marker of mind wandering of an operator of a technical system such as a vehicle, a robot or a manufacturing apparatus ;
- obtaining a perceptual load signal representing a level of perceptual load of the operator;
- determining that the operator has undesirable mind wandering based on the neural marker signal and the perceptual load signal.

12. The mind wandering detection method of claim 11, wherein the method is executed in real-time.

13. A computer program set including instructions for executing the steps of the method of claim 11 or 12 when said program set is executed by at least one computer.

14. A recording medium readable by at least one computer and having recorded thereon at least one computer program including instructions for executing the steps of the method of claim 11 or 12.

## Patentansprüche

1. Vorrichtung zum Detektieren mangelnder Aufmerksamkeit (20) für ein technisches System, wie etwa ein Fahrzeug (10), einen Roboter oder ein Herstellungsgerät, die konfiguriert ist zum:
- Erzielen eines neuronalen Markersignals, das ein Niveau eines metabolischen neuronalen Markers von mangelnder Aufmerksamkeit eines Bedieners des technischen Systems darstellt;
- Erzielen eines Wahrnehmungsbelastungssignals, das ein Niveau einer Wahrnehmungsbelastung des Bedieners darstellt;
- Bestimmen, dass der Bediener eine unerwünschte mangelnde Aufmerksamkeit aufweist, basierend auf dem neuronalen Markersignal und dem Wahrnehmungsbelastungssignal.

2. Vorrichtung zum Detektieren mangelnder Aufmerksamkeit nach Anspruch 1, das dazu konfiguriert ist, zu bestimmen, dass der Bediener eine unerwünschte mangelnde Aufmerksamkeit aufweist, wenn das Niveau des neuronalen Markers größer oder gleich einer ersten Schwelle ist, und das Niveau der Wahrnehmungsbelastung größer oder gleich einer zweiten Schwelle ist.

3. Vorrichtung zum Detektieren mangelnder Aufmerksamkeit nach Anspruch 1 oder 2, die ferner dazu konfiguriert ist, ein Warnsignal auszugeben.

4. Vorrichtung zum Detektieren mangelnder Aufmerksamkeit nach Anspruch 3, die dazu konfiguriert ist, das Warnsignal auszugeben, falls bestimmt wurde, dass die unerwünschte mangelnde Aufmerksamkeit eine Zeit lang anhält, die länger als eine voreingestellte Zeit ist.

5. Vorrichtung zum Detektieren mangelnder Aufmerksamkeit nach einem der Ansprüche 1 bis 4, wobei der metabolische neuronale Marker ein Oxidationszustand eines Cytochrom-c-Oxidase-Enzyms ist.

6. Vorrichtung zum Detektieren mangelnder Aufmerksamkeit nach einem der Ansprüche 1 bis 5, wobei der neuronale Marker ein Marker innerhalb eines präfrontalen Kortexes des Bedieners, wahlweise innerhalb des medialen präfrontalen Kortexes des Bedieners, ist.

7. System (12) zum Detektieren mangelnder Aufmerksamkeit, umfassend die Vorrichtung (20) zum Detektieren mangelnder Aufmerksamkeit nach einem der Ansprüche 1 bis 6, eine neuronale Markersensorvorrichtung (22), die dazu konfiguriert ist, das Niveau des metabolischen neuronalen Markers von mangelnder Aufmerksamkeit zu erfassen und das neuronale Markersignal an die Vorrichtung (20) zum Detektieren mangelnder Aufmerksamkeit auszugeben, und eine Sensorvorrichtung (24) für Wahrnehmungsbelastung, die dazu konfiguriert ist, das Niveau von Wahrnehmungsbelastung zu erfassen und das Wahrnehmungsbelastungssignal an die Vorrichtung (20) zum Detektieren mangelnder Aufmerksamkeit auszugeben.

8. System zum Detektieren mangelnder Aufmerksamkeit nach Anspruch 7, wobei die neuronale Markersensorvorrichtung (22) ein breitbandiges Nahinfrarot-Spektroskop umfasst.

9. System zum Detektieren mangelnder Aufmerksamkeit nach Anspruch 7 oder 8, wobei die Sensorvorrichtung (24) für Wahrnehmungsbelastung eine Kamera (26), die dazu konfiguriert ist, ein Blickfeld des Bedieners abzudecken, und einen Prozessor (28), der mit der Kamera (26) verbunden ist, um mindestens ein Bild von der Kamera (26) zu empfangen und das Niveau der Wahrnehmungsbelastung aus dem mindestens einen Bild zu berechnen, umfasst.

10. Fahrzeug (10), umfassend die Vorrichtung (20) zum Detektieren mangelnder Aufmerksamkeit nach einem der Ansprüche 1 bis 6 oder das System (12) zum Detektieren mangelnder Aufmerksamkeit nach einem der Ansprüche 7 bis 9.

11. Computerumgesetztes Verfahren zum Detektieren mangelnder Aufmerksamkeit, umfassend:
- Erzielen eines neuronales Markersignals, das ein Niveau eines metabolischen neuronalen Markers der mangelnden Aufmerksamkeit eines Bedieners des technischen Systems, wie etwa eines Fahrzeugs, eines Roboters oder eines Herstellungsgeräts, darstellt;
- Erzielen eines Wahrnehmungsbelastungssignals, das ein Niveau der Wahrnehmungsbelastung des Bedieners darstellt;
- Bestimmen, dass der Bediener eine unerwünschte mangelnde Aufmerksamkeit aufweist, basierend auf dem neuronalen Markersignal und dem Wahrnehmungsbelastungssignal.

12. Verfahren zum Detektieren mangelnder Aufmerksamkeit nach Anspruch 11, wobei das Verfahren in Echtzeit ausgeführt wird.

13. Computerprogrammsatz, der Anweisungen umfasst, um die Schritte des Verfahrens nach Anspruch 11 oder 12 auszuführen, wenn der Programmsatz von mindestens einem Computer ausgeführt wird.

14. Aufzeichnungsmedium, das von mindestens einem Computer lesbar ist, und auf dem mindestens ein Computerprogramm aufgezeichnet ist, das Anweisungen zum Ausführen der Schritte des Verfahrens nach Anspruch 11 oder 12 umfasst.

## Revendications

1. Dispositif de détection de vagabondage mental (20) destiné à un système technique tel qu'un véhicule (10), un robot ou un appareil de fabrication, configuré pour :
- obtenir un signal de marqueur neuronal représentant un niveau d'un marqueur neuronal métabolique de vagabondage mental d'un opérateur du système technique ;
- obtenir un signal de charge perceptive représentant un niveau de charge perceptive de l'opérateur ;
- déterminer que l'opérateur présente un vagabondage mental indésirable sur la base du signal de marqueur neuronal et du signal de charge perceptive.

2. Dispositif de détection de vagabondage mental selon la revendication 1, configuré pour déterminer que l'opérateur présente un vagabondage mental indésirable lorsque le niveau du marqueur neuronal est supérieur ou égal à un premier seuil et que le niveau de charge perceptive est supérieur ou égal à un deuxième seuil.

3. Dispositif de détection de vagabondage mental selon la revendication 1 ou 2, configuré en outre pour émettre un signal d'avertissement.

4. Dispositif de détection de vagabondage mental selon la revendication 3, configuré pour émettre le signal d'avertissement s'il est déterminé que le vagabondage mental indésirable dure plus longtemps qu'une période prédéfinie.

5. Dispositif de détection de vagabondage mental selon l'une quelconque des revendications 1 à 4, dans lequel le marqueur neuronal métabolique est un état d'oxydation d'enzyme de cytochrome c-oxydase.

6. Dispositif de détection de vagabondage mental selon l'une quelconque des revendications 1 à 5, dans lequel le marqueur neuronal est un marqueur situé dans le cortex préfrontal de l'opérateur, facultativement dans le cortex préfrontal médian de l'opérateur.

7. Système de détection de vagabondage mental (12), comprenant le dispositif de détection de vagabondage mental (20) selon l'une quelconque des revendications 1 à 6, un dispositif capteur de marqueur neuronal (22) configuré pour acquérir le niveau du marqueur neuronal métabolique de vagabondage mental et pour émettre le signal de marqueur neuronal vers le dispositif de détection de vagabondage mental (20), et un dispositif capteur de charge perceptive (24) configuré pour acquérir le niveau de charge perceptive et pour émettre le signal de charge perceptive vers le dispositif de détection de vagabondage mental (20).

8. Système de détection de vagabondage mental selon la revendication 7, dans lequel le dispositif capteur de marqueur neuronal (22) comprend un spectroscope à infrarouge proche à large bande.

9. Système de détection de vagabondage mental selon la revendication 7 ou 8, dans lequel le dispositif capteur de charge perceptive (24) comprend une caméra (26) configurée pour couvrir un champ de vision de l'opérateur, et un processeur (28) connecté à la caméra (26) pour recevoir au moins une image provenant de la caméra (26) et calculer le niveau de charge perceptive à partir de ladite au moins une image.

10. Véhicule (10) comprenant le dispositif de détection de vagabondage mental (20) selon l'une quelconque des revendications 1 à 6 ou le système de détection de vagabondage mental (12) selon l'une quelconque des revendications 7 à 9.

11. Procédé de détection de vagabondage mental mis en œuvre par ordinateur, comprenant les étapes suivantes :
- obtenir un signal de marqueur neuronal représentant un niveau d'un marqueur neuronal métabolique de vagabondage mental d'un opérateur d'un système technique tel qu'un véhicule, un robot ou un appareil de fabrication ;
- obtenir un signal de charge perceptive représentant un niveau de charge perceptive de l'opérateur ;
- déterminer que l'opérateur présente un vagabondage mental indésirable sur la base du signal de marqueur neuronal et du signal de charge perceptive.

12. Procédé de détection de vagabondage mental selon la revendication 11, dans lequel le procédé est exécuté en temps réel.

13. Ensemble de programmes informatiques comportant des instructions pour exécuter les étapes du procédé selon la revendication 11 ou 12 lorsque ledit ensemble de programmes est exécuté par au moins un ordinateur.

14. Support d'enregistrement lisible par au moins un ordinateur et sur lequel est enregistré au moins un programme informatique comportant des instructions pour exécuter les étapes du procédé selon la revendication 11 ou 12.
